# EUROPÄISCHE PATENTANMELDUNG

(11) **EP 2 210 870 A1**
(43) Veröffentlichungstag der Anmeldung: **28.07.2010**
(21) Anmeldenummer: 09151208.7
(22) Anmeldetag: 23.01.2009
(51) Int. Cl.: C07C 67/333, C07C 51/367, C07C 51/373, C07C 45/51, C07C 47/02, C07C 59/42, C07C 59/147, C07C 69/732, C07C 69/716, C07C 29/32

(54) **Hydroxy- und aldehydfunktionale Verbindungen**

(71) Anmelder: Evonik Degussa GmbH, 45128 Essen (DE)
(72) Erfinder: Meier, Michael A. R., 5223 DH, Hertogenbusch (NL); Dr. Rybak, Anastasiya, 79211 Denzligen (DE); Geisker, Dominik, 26725 (DE)

(57) **Zusammenfassung**

Verfahren zur Herstellung einer hydroxy- und aldehydfunktionale Verbindungen enthaltenden Mischung durch Kreuz-Metathesereaktion mindestens einer mindestens einfach ungesättigten Fettsäure oder einem mindestens einem mindestens einfach ungesättigten Fettsäurederivat mit einer olefinischen Verbindung, die mindestens eine Hydroxygruppe und mindestens eine C-C-Doppelbindung aufweist, in Gegenwart eines Metathesekatalysators bei einer Temperatur von höchstens 180 °C.

## Beschreibung

Die vorliegende Erfindung betrifft ein Verfahren zur Herstellung einer hydroxy- und aldehydfunktionale Verbindungen enthaltenden Mischung.

Verfahren zur Herstellung einer Mischung enthaltend hydroxy- und aldehydfunktionale Verbindungen sind bekannt und entstehen beispielsweise bei der Reduktion von Carbonsäurederivaten oder der Oxidation von Alkoholen.

Aus dem Artikel von Marc L. Snapper et al. "Preparation of Aliphatic Ketones through a Ruthenium-Catalyzed Tandem Cross-Metathesis/Allylic Alcohol Isomerization" Organic Letters, S.2603-2606 (2006) ist die Umsetzung bicyclischer Verbindungen unter ringöffnender Kreuz-Metathese (ROCM = ring opening cross metathesis) mit Z-1,4-Butendiol bekannt, die sowohl zum hydroxy- als auch zum aldehydfunktionalen Reaktionsprodukt reagieren. Des Weiteren ist die Umsetzung von α,β-ungesättigten 4-Hydroxyalkoholen, die in 1- und 4-Position funktionalisiert sind, zu den entsprechenden Ketonen offenbart.

Nachteilig ist bei diesem Verfahren, dass zur Isomerisierung eine Temperatur von 200 °C notwendig ist, und dass signifikante Mengen überreduzierter, d.h. gesättigter Hydroxy-Verbindungen, entstehen. Die Verwendung von Fettsäuren oder deren Derivaten ist nicht erwähnt.

Metathese-Reaktionen werden umfangreich im Rahmen chemischer Synthesen eingesetzt, z.B. in Form von Ringschlussmetathesen (RCM = ring closing metathesis), Kreuzmetathesen (CM = cross metathesis), oder Ringöffnungsmetathese Polymerisation (ROMP = ring opening metathesis polymerization). Anwendung finden Metathese-Reaktionen beispielsweise zur Olefin-Synthese, zur Depolymerisation ungesättigter Polymere und zur Synthese von telechelen Polymeren.

Die Selbstmetathese von Allylalkohol, Allylcyanid und deren Derivaten mit gleichzeitiger Isomerisierung ist darüber hinaus aus dem Artikel von Wagener et al. (J. Mol. Catal. A, Chemical 2003, 194, 69-78) bekannt. Eine neben der Selbstmetathese stattfindende Isomerisierung konnte durch bestimmte Lösungsmittel unterdrückt werden. Kreutzmetathesen wurden nicht offenbart.

Aufgabe der vorliegenden Erfindung ist die Bereitstellung eines Verfahrens zur Herstellung von hydroxy- und aldehydfunktionalen Fettsäurederivaten ohne dass signifikante Mengen (weniger als 5 Gew.-%) an gesättigten Hydroxyverbindungen entstehen.

Die Aufgabe wird gelöst durch ein Verfahren zur Herstellung einer hydroxy- und aldehydfunktionale Verbindungen enthaltenden Mischung durch Kreuz-Metathesereaktion mindestens einer mindestens einfach ungesättigten Fettsäure oder einem mindestens einfach ungesättigten Fettsäurederivat mit einer olefinischen Verbindung, die mindestens eine Hydroxygruppe und mindestens eine C-C-Doppelbindung aufweist, in Gegenwart eines Metathesekatalysators bei einer Temperatur von höchstens 180 °C.

Unter dem Begriff "mindestens einfach ungesättigte Fettsäure" wird hier und im Folgenden eine ungesättigte Carbonsäure verstanden, die einen Kohlenwasserstoffrest mit 1 bis 50 C-Atomen aufweist. Bevorzugte Fettsäuren haben 4 bis 50, insbesondere 6 bis 30 und speziell 8 bis 24 C-Atome wie beispielsweise 12 bis 18 C-Atome. Sie können natürlichen oder synthetischen Ursprungs sein. Besonders bevorzugt sind sie natürlichen Ursprungs und bieten eine ideale Ausgangsplattform auf Basis nachwachsender Rohstoffe. Sie können Substituenten wie beispielsweise Halogenatome, halogenierte Alkylreste, Cyano-, Hydroxyalkyl-, Methoxy-, Nitril-, Nitro-, und/oder Sulfonsäuregruppen tragen mit der Maßgabe, dass diese unter den Reaktionsbedingungen stabil sind und keine Nebenreaktionen wie beispielsweise Eliminierungsreaktionen eingehen. Bevorzugt können die mindestens einfach ungesättigten Kohlenwasserstoffreste linear, verzweigt oder zyklisch sein. Bevorzugt befindet sich keine Doppelbindung in α,β-Position zur Carboxylgruppe.

Als ungesättigte Fettsäuren eignen sich für das erfindungsgemäße Verfahren 3-Hexensäure (Hydrosorbinsäure), trans-2-Heptensäure, 2-Octensäure, 2-Nonensäure, cis- und trans-4-Decensäure, 9-Dexensäure (Caproleinsäure), 10-Undecensäure (Undecylensäure), cis-4-Dodecensäure (Lindersäure), Tridecensäure, cis-9-Tetradecensäure (Myristoleinsäure), Pentadecensäure, cis-9-Hexadecensäure (cis-9-Palmitoleinsäure), trans-9-Hexadecensäure (trans-9-Palmitoleinsäure), Delta-7-cis,10-cis-Hexadecadiensäure, 9-Heptadecensäure, cis-6-Octadecensäure (Petroselinsäure), trans-6-Octadecensäure (Petroselaidinsäure), cis-9-Octadecensäure (Ölsäure), trans-9-Octadecensäure (Elaidinsäure), cis-11-Octadecensäure, trans-11-Octadecensäure (Vaccensäure), cis-5-Eicoseinsäure, cis-9-Eicoseinsäure (Gadoleinsäure), cis-11-Eicoseinsäure (Gondosäure), cis-11,cis-14-Eicosadiensäure, cis-5,cis-8,cis-11,cis-14-Eicosatetraensäure (Arachidonsäure), cis-11-Docosenoinsäure (Cetoleinsäure), cis-13-Docoseinsäure (Erucasäure), trans-13-Docosensäure (Brassidinsäure), cis-15-Tetracosensäure (Selacholeinsäure oder Nervonsäure), cis-17-Hexacosensäure (Ximensäure) und cis-21-Triacontensäure (Lumequesäure), wie auch 2,4-Hexadiensäure (Sorbinsäure), cis-9-cis-12-Octadecadiensäure (Linoleinsäure), cis-9-cis-12-cis-15-Octadecatriensäure (alpha-Linolensäure), cis-6-cis-9-cis-11-Octadecatriensäure (gamma-Linolensäure), Eleostearinsäure, 12-Hydroxy-cis-9-octadecensäure (Ricinolsäure), 9Z,12S,13R-Epoxy-9-octadecensäure (Vernolsäure), cis-5-Deocosensäure, cis-5,13-Docosadiensäure und ähnliche Säuren.

Als Edukte eignen sich auch ungesättigte Fettsäuren mit 10 bis 22 Kohlenstoffatomen, die in Form ihrer technischen Gemische mit anderen Fettsäuren auf Basis von nachwachsenden natürlichen Rohstoffen, z. B. aus Sonnenblumenöl, Rapsöl, Sojaöl, Jatrophaöl, Rizinusöl, Tallöl oder Fischöl, erhalten werden können. Diese mehrfach ungesättigten Fettsäuren werden, wie in der Fettchemie üblich, im Allgemeinen nicht in Form ihrer reinen Verbindungen, sondern in Form ihrer technischen Gemische zur Herstellung der Produkte der Erfindung eingesetzt. Die vorgenannten Fettsäuren werden bevorzugt nicht nur als solche, sondern auch in Form ihrer Ester mit C1-C36-Alkanolen (Alkoholen), insbesondere mit C1-C4-Alkanolen, eingesetzt. Typische Beispiele für derartige Alkanole zur Bildung von Estern mit den vorgenannten Fettsäuren sind Methanol, Ethanol, *n*- bzw. *iso*-Propanol, *n*- bzw. *iso*-Butanol, *n*- bzw. *iso*-Pentanol, *n*- bzw. *iso*-Hexanol, *n*- bzw. *iso*-Octanol, *n*- bzw. *iso*-Decanol, n- bzw. *iso*-Dodecanol, *n*- bzw. *iso*-Tetradecanol, *n*- bzw. *iso*-Hexadecanol, *n*- bzw. iso-Octadecanol sowie höhere bzw. verzweigte Fettalkohole bzw. Fettalkoholderivate mit bis zu 36 Kohlenstoffatomen, z. B. C36-Guerbetalkohole. Die ungesättigten Fettsäuren können auch als Ester mehrwertiger Alkohole, wie Glycerin, Glykol, Pentaerythritol, bis-Pentaerythritol eingesetzt werden. Auch können die Fettsäuren in Form Ihrer Amide, Nitrile, reduzierten Alkohole und anderen Derivaten eingesetzt werden.

Ganz besonders bevorzugt ist der entsprechende Ester mit Methanol, aus dem gemäß dem erfindungsgemäßen Verfahren wichtige Ausgangsprodukte für die Herstellung von Hochleistungspolymeren (Polyamide, Polyester etc.) erhalten werden.

Ebenso wird unter dem Begriff "mindestens einfach ungesättigtes Fettsäurederivat" auch der entsprechende Alkohol verstanden.

Unter dem Begriff "hydroxyfunktionale Fettsäurederivate" wird hier und im Folgenden eine Verbindung verstanden, die einer der zuvor genannten Fettsäurederivate entspricht und zusätzlich mindestens eine OH Gruppe enthält. Die hydroxyfunktionalen Fettsäurederivate können darüber hinaus substituiert sein und beispielsweise eine oder mehrere funktionelle Gruppen wie z.B. Ester, Amid, Nitril, Aldehyd, Keton etc. aufweisen. Sie können auch Substituenten wie beispielsweise Halogenatome, halogenierte Alkylreste, Cyano-, Hydroxyalkyl-, Methoxy-, Nitril-, Nitro-, und/oder Sulfonsäuregruppen tragen mit der Maßgabe, dass diese unter den Reaktionsbedingungen stabil sind und keine Nebenreaktionen wie beispielsweise Eliminierungsreaktionen eingehen.

Unter dem Begriff "aldehydfunktionale Fettsäurederivate" wird hier und im Folgenden eine Verbindung verstanden, die einer der zuvor genannten Fettsäuren entspricht und zusätzlich mindestens eine Aldehyd- und/oder Keto-Gruppe aufweist.

Unter dem Begriff "mindestens einfach ungesättigtes Fettsäurederivat" wird eine Verbindung verstanden, die aus der Reaktion mit einer Fettsäure erhalten wird und einen ungesättigten Kohlenwasserstoffrest aufweist.

Ein Beispiel für ein entsprechendes Fettsäurederivat ist ein ungesättigter Fettsäureester. Das Alkoholsegment des Fettsäureesters kann jeder Monohydroxy-, Dihydroxy- oder Polyhydroxyalkohol sein, der zur Kondensation mit einer ungesättigten Fettsäure, um einen Ester zu bilden, in der Lage ist. In Saatölen ist das Alkoholsegment Glycerin, ein Trihydroxyalkohol. Falls erwünscht, können die Glyceride durch Umesterung in Fettsäureester niedriger Alkohole umgewandelt werden, die einfacher getrennt werden können oder für nachfolgende chemische Prozesse geeignet sind. Typischerweise enthält der Alkohol, der in der Umesterung verwendet wird, wenigstens ein Kohlenstoffatom. Typischerweise enthält der Alkohol weniger als etwa 15 Kohlenstoffatome, vorzugsweise weniger als etwa 12 Kohlenstoffatome, besonders bevorzugt weniger als etwa 10 Kohlenstoffatome, und noch mehr bevorzugt, weniger als ungefähr 8 Kohlenstoffatome. Die Kohlenstoffatome in dem Alkoholsegment können in einer geraden Kette oder einer verzweigten Struktur angeordnet sein und können mit einer Vielzahl von Substituenten, wie zum Beispiel denen, die vorhergehend hierin im Zusammenhang mit den Olefinreaktanten offenbart sind, substituiert sein, einschließlich der oben erwähnten Alkyl-, Cycloalkyl-, monocyclischen aromatischen, Arylalkyl-, Alkylaryl-, Hydroxyl-, Halogen-, Nitro-, Carbonsäure-, Ether-, Ester-, Acyl- und Amidsubstituenten. Vorzugsweise ist das Alkoholsegment des ungesättigten Fettsäureesters Glycerin oder ein geradkettiges oder verzweigtes C1-C8 Alkanol. Es ist am meisten bevorzugt, wenn der Alkohol ein C1-C3; Alkanol ist, geeignete Beispiele davon beinhalten Methanol, Ethanol und Propanol.

Weitere Fettsäurederivate, die sich für das erfindungsgemäße Verfahren eignen, sind Fettalkohole, die durch Reduktion aus den Fettsäuren oder Fettsäurederivaten gewonnen werden; veresterte Fettalkohole; Fettamide, Fettamine(primäre, sekundär, tertiär), Fettnitrile; langkettige ungesättigte Verbindungen welche eine funktionelle Gruppe enthalten. Beispiele für funktionelle Gruppen sind alle geeignete, dem Fachmann an sich bekannten funktionelle Gruppen, wie beispielsweise Halogenatome, halogenierte Alkylreste, Cyano-, Hydroxyalkyl-, Methoxy-, Nitril-, Nitro-, Amino-, Hydroxy- und/oder Sulfonsäuregruppen.

Unter dem Begriff "olefinische Verbindung, die mindestens eine Hydroxygruppe und mindestens eine C-C-Doppelbindung aufweist" wird hier und im Folgenden eine solche Verbindung verstanden, die einen ungesättigten Kohlenwasserstoffrest mit 3 bis 10 Kohlenstoffatomen aufweist.

Beispiele hierfür sind Prop-2-en-1-ol (Allylalkohol), But-3-en-1-ol, Pent-4-en-1-ol, Hex-5-en-1-ol, Hept-6-en-1-ol, Oct-7-en-1-ol, Non-8-en-1-ol, Dec-9-en-1-ol. Beispiele für ungesättigte Diole sind But-2-en-1,4-diol, Hex-3-en-1,6-diol, Dec-5-en-1,10-diol und ähnliche.

Ganz besonders für das erfindungsgemäße Verfahren geeignet ist Allylalkohol, But-3-en-1-ol, und 1,4-But-2-en-diol.

Unter dem Begriff "Metathesekatalysator" wird hier und im Folgenden eine Verbindung verstanden, die die folgende Struktur aufweist 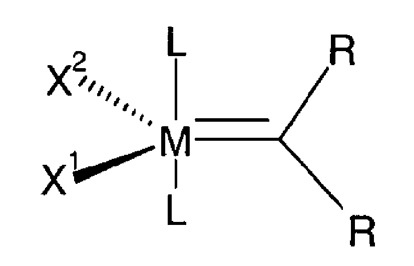 in der M Osmium oder Ruthenium bedeutet, R für gleiche oder verschiedene organische Reste mit großer struktureller Variationsbreite steht, X¹ und X² anionische Liganden bedeuten und L gleiche oder verschiedene, neutrale Elektronen-Donoren darstellt. Unter dem gängigen Begriff "anionische Liganden" werden in der Literatur für derartige Metathese-Katalysatoren immer solche Liganden verstanden, die, wenn man sie als vom Metall-Zentrum entfernt betrachtet, bei geschlossener Elektronenschale negativ geladen sind.

Solche Katalysatoren sind beispielsweise aus der WO-A-96/04289 und WO-A-97/06185 bekannt.

Aus WO-A-00/71554 ist ferner eine Gruppe von Katalysatoren bekannt, die in der Fachwelt als "Grubbs(II)Katalysatoren" bezeichnet werden: 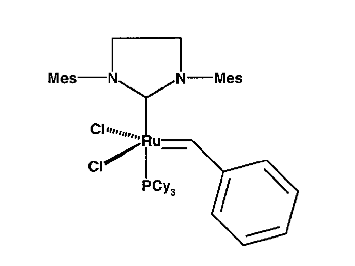 Weitere Metathesekatalysatoren sind beispielsweise in WO 99/51344, WO 00/15339, WO 00/71554, WO 02/079126, WO 02/79127, WO 02/083742 und z.B. in Angew. Chem. 1995, 107(18), 2179; J. Am. Chem. Soc. 1995, 117, 5503; J. Am. Chem. Soc. 1996, 118, 100; Chem. Eur. J. 2001, 7, 3236; Organometallics 2000, 19(11), 2055; J. Am. Chem. Soc. 1999, 121, 2674; Organic Letters 1999, 1(6), 953; J. Am. Chem. Soc. 2003, 125, 10103; J. Am. Chem. Soc. 2003, 125, 2546; J. Am. Chem. Soc. 2001, 123, 6543 beschrieben. Weitere Beispiele für Grubbs-(II)-Katalysatoren sind beispielsweise in WO 02/14376, WO 03/011875, WO 03/044060, WO 04/035596, US 6,620,955 und z.B. in J. Am. Chem. Soc. 1999, 121, 791; J. Am. Chem. Soc. 2000, 122, 8168; Tetrahedron Letters 2000, 41, 9973; Tetrahedron 2003, 59, 6545; Synlett 2001, 3, 430; Adv. Synth. Catal. 2003, 345, 572; Synlett 2004, 4, 667; Eur. J. Org. Chem. 2003, 963; Chem. Eur. J. 2004, 10, 2029 beschrieben.

Besonders geeignet für erfindungsgemäße Verfahren sind Benzyliden-bis(tri-cyclohexylphosphin)dichlororuthenium (Grubbs Catalyst 1^{st} Generation), 1,3-Bis-(2,4,6-trimethylphenyl)-2-(imidazolidinyliden)(dichlorophenylmethylen)(tricyclohexylphosphin)ruthenium (Grubbs Catalyst 2^{nd} Generation), (1,3-Bis-(2,4,6-trimethylphenyl)-2-imidazolidinyliden)dichloro(o-isopropoxyphenylmethylen)ruthenium (Hoveyda-Grubbs Catalyst 2^{nd} Generation) und 1,3-Bis-(2,4,6-trimethylphenyl)-4,5-dihydroimidazol-2-yliden[2-(i-propoxy)-5-N,N-dimethylaminosulfonyl)phenyl]methylenruthenium(II)dichlorid (Zannan-Katalysator).

Ganz besonders bevorzugt sind Komplexverbindungen der allgemeinen Formeln

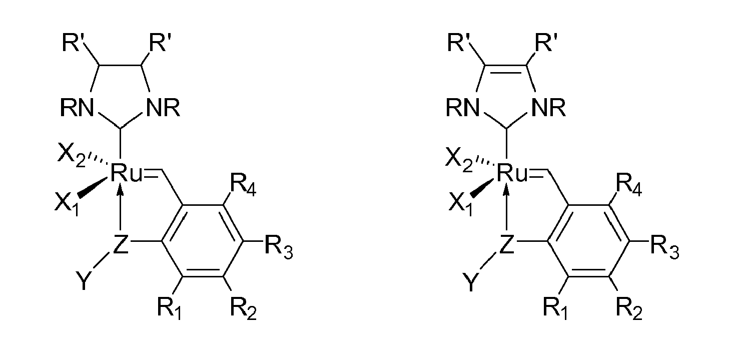

Darin steht
- Z für -O-, -S-, -S(O)- und S(O)₂-,X1 und X2 nehmen die oben angegebenen Reste an,
- Y, R, R' und R₁ bis R₄ sind unabhängig voneinander wählbare Reste aus der Gruppe Wasserstoff, (C₁-C₈)-Alkyl, (C₁-C₈)-Alkoxy, (C₆-C₁₈)-Aryloxy, HO-(C₁-C₈)-Alkyl, (C₂-C₈)-Alkoxyalkyl, (C₆-C₁₈)-Aryl, (C₇-C₁₉)-Aralkyl, (C₃-C₁₈)-Heteroaryl, (C₄-C₁₉)-Heteroaralkyl, (C₁-C₈)-Alkyl-(C₆-C₁₈)-Aryl, (C₁-C₈)-Alkyl-(C₃-C₁₈)-Heteroaryl, (C₃-C₈)-Cycloalkyl, (C₁-C₈)-Alkyl-(C₃-C₈)-Cycloalkyl, (C₃-C₈)-Cycloalkyl-(C₁-C₈)-Alkyl. Außerdem können die Reste R' und R₁ bis R₄ unabhängig voneinander bedeuten: (Cyclo)Alkylthio, (Hetero)Arylthio, Alkyl/Arylsulfonyl Alkyl/Arylsulfinyl, jeweils wahlweise substituiert mit (C₁-C₈)-Alkyl, (C₁-C₈)-Alkoxy, (C₆-C₁₈)-Aryloxy, HO-(C₁-C₈)-Alkyl, (C₂-C₈)-Alkoxyalkyl, (C₆-C₁₈)-Aryl, Perfluoralkyl, Halogen, (C₁-C₈)-Acyloxy, (C₁-C₈)-Acyl (C₁-C₈)-Alkoxycarbonyl, (C₁-C₈)-Alkylsulfonyl oder (C₁-C₈)-Alkylsulfinyl, (C₆-C₁₈)-Arylsulfonyl oder (C₆-C₁₈)-Arylsulfinyl.
- R₁ bis R₄ können ebenfalls eine Nitro-Gruppe, Sulfat, Amin, Ammoniumsalz, Phospat und Phosphoniumsalz bedeuten.
- die Reste R' können mit einem oder mehreren der Reste R₁ bis R₄ in cyclischen Verbindungen miteinander verknüpft vorliegen. Auch der Rest R₁ kann mit dem Rest Y unter Ausbildung einer (hetero)cyclischen Verbindung miteinander verknüpft sein.

Beispiele für die oben genannten Verbindungen sind:

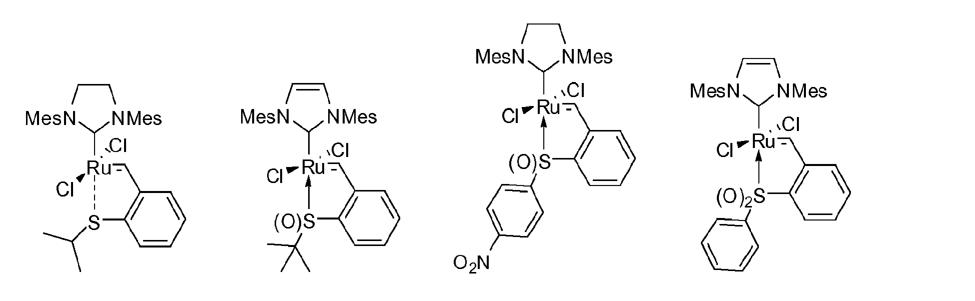

Gemäß einer besonders bevorzugten Ausführungsfom der vorliegenden Erfindung liegt die olefinische Verbindung, die mindestens eine Hydroxygruppe und mindestens eine C-C-Doppelbindung aufweist, im Überschuss vor - bezogen auf die mindestens einfach ungesättigte Fettsäure oder das mindestens einfach ungesättigte Fettsäurederivat. Diese Ausführungsform hat den Vorteil, dass noch weniger Selbstmetathese stattfindet.

Besonders bevorzugt liegt die Temperatur während der Kreuz-Metathesereaktion zwischen 80 und 120 °C. Dieser Temperaturbereich ermöglicht eine hohe Ausbeute an aldehydfunktionalen Verbindungen, die Ausbeute an hydroxyfunktionalen Verbindungen nimmt in diesem Temperaturbereich ab.

Gemäß einem ebenfalls bevorzugten Verfahren liegt die Temperatur während Kreuz-Metathesereaktion zwischen 0 und 60 °C. Dieser Temperaturbereich wiederum ermöglicht eine hohe Ausbeute an hydroxyfunktionalen Verbindungen, während die Ausbeute an aldehydfunktionalen Verbindungen in diesem Temperaturbereich abnimmt.

Eine weitere, ebenfalls besonders bevorzugte Ausführungsform betrifft das sogenannte Zweistufenverfahren. Hierbei wird die Kreuz-Metathesereaktion zuerst bei einer Temperatur zwischen 0 und 60 °C durchgeführt. Die so erhaltene Mischung wird dann auf eine Temperatur zwischen 80 und 120 °C gebracht. Dieses Zweischrittverfahren, d.h. zuerst die Reaktionsführung bei vergleichsweise niedriger Temperatur und das anschließende Aufheizen auf eine deutlich höhere Temperatur, hat den Vorteil, dass mit einer einfachen Änderung der Reaktionsführung die Ausbeute - durch Isomerisierung der hydroxyfunktionalen Verbindung - deutlich in Richtung der aldehydfunktionalen Verbindung verschoben wird, der Anteil an hydroxyfunktionaler Verbindung ist sehr gering und liegt bei weniger als 5 %. Im einfachsten Fall werden der Reaktionsmischung weder Verbindungen noch Lösemittel hinzugegeben oder entzogen, lediglich die Temperatur wird möglichst schnell auf den zuvor genannten Bereich erhöht.

In einer weiteren, bevorzugten Ausführungsform der vorliegenden Erfindung wird die Kreuz-Metathesereaktion in Gegenwart mindestens eines Lösemittels durchgeführt.

Prinzipiell können alle für die Metathese-Reaktion geeigneten Lösungsmittel ausgewählt werden, die den eingesetzten Katalysator nicht desaktivieren und die Reaktion auch nicht in irgendeiner anderen Weise negativ beeinflussen. Bevorzugte Lösungsmittel umfassen, sind aber nicht begrenzt auf, Dichlormethan, Benzol, Toluol, Methylethylketon, Aceton, Tetrahydrofuran, Tetrahydropyran, Dioxan, Cyclohexan, Methanol, Ethanol, Propanol, iso-Propanol, Butanol, "alle Alkohole" und alle aliphatischen und alizyklischen Kohlenwasserstoffe unterschiedlicher Kohlenstoffzahl, sowie alle halogenierten Kohlenwasserstoffe. In manchen Fällen, wenn die olefinische Verbindung, die mindestens eine Hydroxygruppe und mindestens eine C-C-Doppelbindung aufweist, selbst als Lösungsmittel fungieren kann, so z.B. bei But-3-en-1-ol oder But-3-en-1,4-diol, kann auch auf den Zusatz eines weiteren zusätzlichen Lösungsmittels verzichtet werden.

Nicht als Lösemittel im vorgenannten Sinne werden Amine, Hydroxythiole, Dithiole, Thioacetale sowie gesättigte organische Verbindungen mit mindestens zwei Hydroxylgruppen verstanden, wie beispielsweise Ethylenglykol, Glycerin, 1,3-Propandiol oder Propylenglykol, da ungesättigte Di- oder Polyole bei mehr als 60 °C nicht nur als Lösemittel, sondern auch als Reaktand mit Schutzgruppenfunktion fungieren und mit der entstehenden aldehydfunktionalen Verbindung zum entsprechenden cyclischen Vollacetal reagieren.

Besonders bevorzugt wird ein solches Lösemittel ausgewählt, welches ein Dipolmoment zwischen 0 und 5 Debye aufweist. Ganz besonders bevorzugt ist ein Dipolmoment zwischen 0 und 2,5 Debye.

Beispiele für ein solches Lösemittel sind Dichlormethan (1,60 D), 1,2-Dichlorethan (1,48 D), Toluol (0,375 D), THF (1,75 D, gemessen als 25%ige Lösung in Benzol), 1-Butanol (1,66 D), 1,2-Diethoxyethan (1,99, gemessen als 20%ige Lösung in Benzol), 1,4-Dioxan (0 D).

Ganz besonders bevorzugt sind Tetrahydrofuran, *iso*-Propanol und 1-Butanol.

In einer weiteren erfindungsgemäßen Ausführungsform wird die Kreuz-Metathesereaktion in homogener Phase durchgeführt. Hierunter wird verstanden, dass weder eine Phasentrennung vorhanden ist, noch dass die Reaktanden in einer Suspension oder Dispersion vorliegen.

Die homogene Reaktionsführung hat den Vorteil, dass erhöhte Kreuzmetathese-Ausbeuten beobachtet werden.

Die Menge des Metathese-Katalysators bezogen auf die eingesetzte mindestens einfach ungesättigte Fettsäure oder das mindestens einfach ungesättigte Fettsäurederivat hängt von der Natur sowie der katalytischen Aktivität des speziellen Katalysators ab. Die Menge an eingesetztem Katalysator beträgt üblicherweise 1 bis 1.000 ppm Edelmetall, bevorzugt 2 bis 500 ppm, insbesondere 5 bis 250 ppm, bezogen auf die eingesetzte Fettsäure oder das Fettsäurederivat.

Die Reaktionszeit hängt von einer Reihe von Faktoren ab, beispielsweise vom Typ der verwendeten, einfach ungesättigte Fettsäure oder des mindestens einfach ungesättigten Fettsäurederivats, der Art des Katalysators, der verwendeten Katalysator-Konzentration und der Reaktionstemperatur. Typischerweise ist die Reaktion innerhalb von drei Stunden unter normalen Bedingungen beendet. Der Fortschritt der Metathese kann durch Standard-Analytik überwacht werden, z.B. durch GC/MS- oder HPLC/MS-Messungen.

Zur Verbesserung der Isomerisierung, d.h. zur Verschiebung der Produktverhältnisse in Richtung aldehydfunktioneller Verbindung, können der Reaktionsmischung zusätzlich dem Fachmann an sich bekannte Additive hinzugegeben werden, wie beispielsweise Hydride oder Basen.

Es ist aber auch möglich, zur Unterdrückung der Isomerisierung, d.h. einer Verschiebung des Produktverhältnisses hin zur hydroxyfunktionellen Verbindung, der Reaktionsmischung zusätzlich dem Fachmann an sich bekannte Additive hinzu zu gegeben, wie beispielsweise Phenylphosphorsäure oder Chinone.

Die folgenden Beispiele dienen der Erläuterung der Erfindung, ohne die Erfindung hierauf zu beschränken oder sonst wie einzuschränken.

### 1. Allgemeine Angaben

Lösungsmittel und Chemikalien. Es wurden folgende handelsübliche Lösungsmittel und Chemikalien verwendet: Aceton (99.5 % GPR Rectapur von VWR Int., Art.-Nr. 20065.470), Benzyliden-bis(tricyclohexylphosphin) dichlororuthenium (GI, Grubbs Catalyst 1^{st} Generation von Aldrich, Art.-Nr. 579726), 1,3-Bis-(2,4,6-trimethylphenyl)-2-(imidazolidinyliden)(dichlorophenylmethylen) (tricyclohexylphosphin)ruthenium (GII, Grubbs Catalyst 2^{nd} Generation von Aldrich, Art.-Nr. 569747), (1,3-Bis-(2,4,6-trimethylphenyl)-2-imidazolidinyliden)dichloro(o-isopropoxyphenylmethylen)ruthenium (HGII, Hoveyda-Grubbs Catalyst 2^{nd} Generation von Aldrich, Art.-Nr. 569755), 1,3-Bis-(2,4,6-trimethylphenyl)-4,5-dihydroimidazol-2-yliden[2-(i-propoxy)-5-N,N-dimethylaminosulfonyl)phenyl]methylenruthenium (II) dichlorid (ABCR, 96 % von ABCR, Art.-Nr. AB173300), 1-Butanol (99.9 % von Sigma-Aldrich, Art.-Nr. 537993), 2-Buten-1,4-Diol (≥ 98.0 % von Fluka, Art.-Nr. 03781), 1,2-Diethoxyethan (98% von Alfa Aesar, Art.-Nr. L14283), Diethylether (99 % GPR Rectapur von VWR Int., Art.-Nr. 23809.363), 1,2-Dimethoxyethan (≥ 98.0 % von Fluka, Art.-Nr. 38570), 2,4-Dinitrophenylhydrazin (97 % von Aldrich, Art.-Nr.
D199303), 1,4-Dioxan (wasserfrei, 99.8 % von Aldrich, Art.-Nr. S39401-018), Ethylvinylether (99 % von Aldrich, Art.-Nr. 422177), n-Hexan (95.0 % AnalaR Normapur von VWR Int., Art.-Nr. 24577.367), n-Hexan (≥ 97.0 % Chromasolv^{®} für HPLC von Sigma-Aldrich, Art.-Nr. 34859), Kupfer(II)-sulfat Hydrat (98 % von Aldrich, Art.-Nr. 209201), Methanol (≥ 99.9 % Chromasolv^{®} für LC-MS von Fluka, Art.-Nr. 34966), Natriumhydrid (60%ige Dispersion in Mineralöl von Aldrich, Art.-Nr. 452912), Natriumhydroxid (≥ 99 % von Carl Roth, Art.-Nr. 6771-3), Ölsäuremethylester (99 % SAFC^{™} von Sigma-Aldrich, Art.-Nr. S45393-118), 2-Propanol (99.9 % Chromasolv^{®} plus für HPLC von Sigma-Aldrich, Art.-Nr. 650447), Schiff's Reagens (Fluka, Art.-Nr. 84655), Schwefelsäure (95 - 97 % von Fluka, Art.-Nr. 84718), Tetradecan (olefinfrei, ≥ 99 % von Fluka, Art.-Nr. 87140), Tetrahydrofuran (Rotisolv^{®} für HPLC von Carl Roth, Art.-Nr. 7344.2), Tributylamin (≥ 98.5 % von Aldrich, Art.-Nr. 471313), Undecanal (97 % von Alfa Aesar, Art.-Nr. A16101), trans-2-Undecen-1-ol (96 % von ABCR, Art.-Nr. AB125432), Weinsäure Kalium-Natriumsalz Tetrahydrat (99 % von Sigma-Aldrich, Art.-Nr. 217255), o-Xylol (98.0 % von Fluka, Art.-Nr. 95663). Die Reinigung und Trocknung erfolgte - soweit erforderlich - nach allgemein üblichen Laboratoriumsmethoden.

### Analytische Geräte und Methoden

Dünnschichtchromatographie: Für die dünnschichtchromatographischen Untersuchungen (DC) wurden Kieselgel-Alufolien (Schichtdicke 0.2 mm von Fluka, Art.-Nr. 60800) verwendet. Die Trennungen erfolgten unter Kammersättigung mit Hexan/Diethylether (1 : 1) als Fließmittel auf einer Trennstrecke von 8-10 cm. Die Substanzzonen wurden mit Permanganat-Reagenz visualisiert. DC-Nachweis der Aldehyde erfolgte mit Dinitrophenylhydrazin-Reagenz (Gemisch aus 1 g 2,4-Dinitrophenylhydrazin, 25 mL Ethanol, 8 mL Wasser und 5 mL konz. Schwefelsäure).

Säulenchromatographie: Säulenchromatographische Trennungen wurden an Kieselgel 60, Korngröße 35 - 70 µm (Fluka, Art.-Nr. 60738) durchgeführt. Eine chromatographische Säule mit einer Länge von 40 cm und einem Innendurchmesser von 2 cm wurde verwendet. Als Fließmittel diente das Gemisch Hexan/Diethylether (9 : 1).

Infrarotspektroskopie. IR-Spektren wurden mit einem Bruker EQUINOX 55 FTIR Spektrometer mit ATR-Zelle im Wellenzahlenbereich von 4600 bis 600 cm⁻¹ aufgenommen.

NMR-Spektroskopie: Die ¹H-NMR (¹³C-NMR) Spektren wurden mit einem Bruker AVANCE DPX (¹H: 300 MHz / ¹³C : 75,5 MHz) aufgenommen. Als Standard für die chemische Verschiebung δ diente das Signal des Tetramethylsilans (TMS, δ = 0.00 ppm). Die Kopplungskonstanten (J) sind in Hertz (Hz) angegeben. Die Kopplungsmuster wurden folgendermaßen abgekürzt: s = Singulett; d = Dublett; t = Triplett; q = Quartett; m = Multiplett.

Gaschromatographie (GC): Gaschromatographische Untersuchungen erfolgten mit einem GC-2010 (Shimadzu) Chromatographen, ausgestattet mit Autosampler, Flammenionisationsdetektor (FID) sowie GC-Kapillarsäule Stabilwax^{®} (30m x 0.25mm x 0.25 µm, Restek, Art.-Nr. 10623). Messungen wurden im Split-Modus (Split-Rate 1:45) mit Wasserstoff als Trägergas (Flußrate 1 mL/min, lineare Trägergasgeschwindigkeit 31.4 cm/sec) durchgeführt. Temperaturprogramm für GC-Ofen: Starttemperatur 95 °C, für 1 min halten; mit 8 °C/min bis auf 220 °C heizen, für 4 min halten; mit 8 °C/min bis auf 248 °C heizen, für 7 min halten. Detektor- und Injektortemperaturen betrugen 250 und 220 °C.

GC-MS (EI): GC-MS EI-Messungen (70 eV) wurden mit einer VARIAN 3900 GC mit Saturn 2100T lon-Trap Massendetektor sowie GC-Kapillarsäule FactorFour^{™} VF-5ms (30m x 0.25mm x 0.25 µm, Varian, Art.-Nr. CP8944) durchgeführt. Messungen erfolgten im Massenbereich von 40 bis 650 m/z mit der Rate von 1.0 scan/s. Messungen wurden mit und ohne Split (Split-Rate 1:50) mit Helium als Trägergas (Flußrate 1 mL/min) durchgeführt. Temperaturprogramm für GC-Ofen: Starttemperatur 95 °C, für 1 min halten; mit 15 °C/min bis auf 220 °C heizen, für 4 min halten; mit 15 °C/min bis auf 300 °C heizen, für 2 min halten. Injektortemperatur betrug 250 °C, Injektionsvolumen 1 µL. Die Probelösungen wurden in n-Hexan (HPLC Reinheitsgrad) mit der Konzentration 1 mg/mL vorbereitet.

Elektrospray-lonisation (ESI-positiv) Massenspektroskopie: Messungen wurden mit einem VARIAN 500-MS Ion-Trap Spektrometer im Fullscan-Modus (Massenbereich 40-650 m/z) durchgeführt. Analyte wurden in Methanol für LC-MS (Konzentration 1 mg/mL) gelöst und mittels einer Spritzenpumpe direkt appliziert.

Gel Permeation Chromatographie (GPC): Die Bestimmung der Molekulargewichte erfolgte mit einem LC-20AD Chromatograph der Firma Shimadzu, ausgestattet mit SIL-20A Autosampler, CTO-20A Ofen, DGU-20A₃ Degasser und RID-10A Brechungsindexdetektor. Die Messungen erfolgten isokratisch bei 50 °C mit einer PLgel 5µm Mixed-D GPC-Säule (300 mm x 7.5 mm x 5 µm, Polymerlabs, Art.-Nr.
PL1110-6504) mit THF als Eluent (Flußrate 1.0 mL/min). Für die Kalibrierung der Säule wurden lineare Poly(methylmethacrylat)-Standards (Polymer-Kit der Firma Polymer Standards Service PPS, Germany, Mp 102 - 981.000 Da, Art-Nr. PSSmmkith) verwendet.

Nachweis von Aldehyden mit Färbungsreagenzien: Zum qualitativen Nachweis von Aldehyden in den Reaktionsgemischen wurden die Fehlingsche Lösung (nach gängigen Laboratoriumsmethoden frisch hergestellt) und das Schiff' sche Reagenz (Fluka) verwendet.

Ausbeuten: Angegebene Ausbeute - falls nicht anders angegeben - beziehen sich auf die mittels GC-chromatographischen Daten berechneten Produkte.

Glasgeräte und Apparatur: Für die Durchführung der Reaktionen im 1 mL Maßstab wurden Reagenzgläser mit NS14/23-Normschliff und PE-Stopfen (Gesamtvolum 10 mL, von VWR, Art.-Nr. 212-1622) verwendet. Reaktionen wurden im Silikonölbad auf einer Heizplatte mit integriertem Magnetrührer IKA^{®} RET Basic und elektronischem Kontakttermometer IKA^{®} ETS-D5 durchgeführt.

Die Durchführung der Reaktionen im 2 mL Maßstab erfolgte in einer Carousel Reaction Station^{™} RR98072 der Firma Radleys Discovery Technologies.

Die Isomerisierungsversuche unter inerten Atmosphäre wurden in 3 mL-Reaktionsgläser mit Schraubverschluß und Septum (Supelco bei Aldrich, Art.-Nr. 33297) durchgeführt. Das Reaktionsgemisch wurde mit dem Inertgas (PR Stickstoff BIP-X10S von Air Products) durch Septum mittels Kanülen gespült. Das Temperieren der Reaktionsgemische erfolgte in einem Heizblock (Sigma-Aldrich, Art.-Nr. Z210188-1 EA) mit Hilfe einer Heizplatte mit integriertem Magnetrührer IKA^{®} RET Basic und elektronischem Kontaktthermometer IKA^{®} ETS-D5. Für alle anderen Isomerisierungen wurden die gleichen Glasgeräte und Apparatur benutzt, wie für die Reaktionen im 1 mL Maßstab (s. Beschreibung oben).

### 2. Synthesevorschriften

### Allgemeine Vorschriften

Reaktionen im 1 mL Maßstab: Synthesen erfolgten mit 1 mL Ölsäuremethylester unter diversen Reaktionsbedingungen: die Reaktionen wurden ohne Lösungsmittel, sowie in 1-Butanol und o-Xylol durchgeführt. Sämtliche Reaktionen wurden mit zwei verschiedenen Verhältnissen von Butendiol (1 und 3 Äquivalent zu Ölsäuremethylester) und bei zwei verschiedenen Temperaturen (60 und 120 °C) durchgeführt. Drei Ruthenium Metathese Katalysatoren (kommerziell erhältlich von Aldrich) wurden für alle Verhältnisse und Temperaturen getestet.

Allgemeine Methode: A) Route ohne Zusatzstoffe: Im Reaktionsglas wird das Gemisch aus 1 mL (2.95 mmol) Ölsäuremethylester, die entsprechende Menge von 2-Buten-1,4-diol (0.24 mL oder 0.73 mL) und 0.1 mL Tetradecan (interner Standard) vorbereitet und gemischt. Wenn die Reaktion lösungsmittelfrei durchgeführt wird, wird eine Null-Probe für GC-Messung genommen. Bei der Durchführung mit Lösungsmittel wird eine Null-Probe für GC-Messung erst nach Zugabe von 1 mL der entsprechenden Lösungsmittel genommen. Nach der Probenahme wird das Reaktionsgefäß ins temperierte Ölbad plaziert. Das Gemisch wird ca. 2 Minuten mittels eines Magnetrührers gerührt. Nachdem die gewünschte Temperatur des Gemisches erreicht ist, wird der Katalysator (2 mol.%) zugegeben. Das Reaktionsgemisch wird weiter temperiert und gerührt, nach 4 und 24 Stunden Reaktion werden die nächsten GC-Proben für kinetische Bestimmungen genommen. Nach 24 Stunden wird die Reaktion durch Zugabe von 1 mL Ethyl-vinylether gestoppt.

B) Route mit Additiva: Im Reaktionsglas wird das Gemisch aus 1 mL (2.95 mmol) Ölsäuremethylester, die entsprechende Menge von 2-Buten-1,4-diol (0.24 mL oder 0.73 mL) und 0.1 mL Tetradecan (interner Standard) vorbereitet und gemischt. Wenn die Reaktion lösungsmittelfrei durchgeführt wird, wird eine Null-Probe für GC-Messung genommen. Bei der Durchführung im Lösungsmittel wird eine Null-Probe für GC-Messung erst nach Zugabe von 1 mL des entsprechenden Lösungsmittels genommen. Nach der Probenahme wird das Reaktionsglas ins temperierte Ölbad plaziert. Das Gemisch wird ca. 2 Minuten mittel eines Magnetrührers gerührt. Nachdem die gewünschte Temperatur des Gemisches erreicht ist wird der Katalysator (2 mol.%) zugegeben. Das Reaktionsgemisch wird weitere 4 Stunden temperiert und gerührt. Nach 4 Stunden Reaktion wird die nächste GC-Probe genommen. Nach der Probenahme wird das entsprechende Additivum zugegeben. Als Additiva wurden Natriumhydrid und Tributylamin (jeweils 1 und 10 mol.% bezogen auf den Katalysator) verwendet. Nach 24 Stunden wird die letzte GC-Probe genommen und die Reaktion durch Zugabe von 1 mL Ethyl-vinylether gestoppt.

Reaktionen im 2 mL Maßstab: Synthesen erfolgten mit 2 mL Ölsäuremethylester unter folgenden Reaktionsbedingungen: die Reaktionen wurden in verschiedenen Lösungsmittel (Aceton, 1-Butanol, 1,4-Dioxan, 2-Propanol, THF) durchgeführt. Sämtliche Reaktionen wurden mit zwei verschiedenen Verhältnissen von Butendiol (3 und 5 Äquivalente bezogen auf Ölsäuremethylester) und bei zwei verschiedenen Temperaturen (40 und 60 °C) durchgeführt. Ruthenium-basierte Metathese-Katalysatoren (kommerziell erhältlich bei Aldrich bzw. ABCR und Testmuster der Evonik Degussa GmbH) wurden für alle Verhältnisse und Temperaturen getestet. Es wurden keine Additiva verwendet.

Allgemeine Methode: Im Reaktionsgefäß wird das Gemisch aus 2 mL (5.9 mmol) Ölsäuremethylester, die entsprechende Menge von 2-Buten-1,4-diol (1.46 mL oder 2.43 mL), 0.2 mL Tetradecan (interner Standard) und 2 mL des entsprechenden Lösunngsmittels vorbereitet und geschüttelt. Danach wird eine Null-Probe für GC-Messung genommen. Nach der Probenahme wird der entsprechende Katalysator (2 mol.%) zugegeben und das Reaktionsgefäß wird im temperierten Carousel-Reaktor plaziert. Das Gemisch wird 4 Stunden temperiert und weiterhin gerührt, danach wird die letzte GC-Probe für kinetische Bestimmungen genommen. Nach 4 Stunden wird die Reaktion durch Zugabe von 2 mL Ethyl-vinylether gestoppt.

Zur Untersuchung des Zweistufenverfahrens wird ein Modellversuch ausgehend von kommerziell erhältichen trans-2-Undecen-1-ol durchgeführt: Die Synthesen erfolgten mit 0.1 mL trans-2-Undecen-1-ol unter Stickstoff-Atmosphäre und ohne Schutzgas. Die Reaktionen wurden in verschiedenen Lösungsmitteln (1-Butanol, 1,4-Dioxan, 1,2-Dimethoxyethan, 1,2-Diethoxyethan) sowie lösungsmittelfrei durchgeführt. Die verschiedenen Konzentrationen von 2-Undecen-1-ol (Verdünnung 1 : 3, 1 : 10, 1 : 20, 1 : 30, 1 : 50) und verschiedene Temperaturen wurden untersucht. Dabei wurde das trans-2-Undecen-1-ol mit dem Katalysator und dem Lösemittel auf eine Temperatur von maximal 60 °C für eine Dauer von maximal 5 Minuten (zur Verhinderung der Zersetzung zu Butendiol) erhitzt und die so erhaltene Mischung zügig auf verschiedene Temperaturen von mindestens 80 °C erhitzt (beispielsweise durch Transfer der Reaktionsgefäßes in ein vorgeheitztes Ölbad). Ruthenium-basierte Metathese-Katalysatoren (kommerziell erhältlich bei Aldrich bzw. ABCR) wurden für alle Verhältnisse und Temperaturen (80, 100 und 120 °C) getestet.

Die Isomerisierung des 2-Undecen-1-ols zu Undecanal ist eine temperaturabhängige Reaktion. Unter hohen Temperaturen ( > 100 °C ) verläuft die Reaktion sehr schnell (innerhalb von 5 Minuten) mit hoher Konversion. Die maximale Ausbeute des Undecanals wird (unabhängig von der Temperatur) innerhalb der ersten 0.5 Stunden erzielt. Das längere Erhitzen des Gemisches führt zur Zunahme des Umsatzes und zur gleichzeitigen Abnahme der Undecanal-Konzentration infolge Nebenreaktionen (Oligomerisierung, Bildung von Acetalen und Halbacetalen bei der Reaktion im Alkohol als Lösungsmittel).

Allgemeine Methode: A) Isomerisierung unter inerter Atmosphäre: Im Reaktionsglas wird das Gemisch aus 0.1 mL (0.49 mmol) trans-2-Undecen-1-ol und 0.02 mL Tetradecan (interner Standard) vorbereitet und gemischt. Wenn die Reaktion lösungsmittelfrei durchgeführt wird, wird eine Null-Probe für GC-Messung genommen. Bei der Durchführung mit Lösungsmittel wird eine Null-Probe für GC-Messung erst nach Zugabe des entsprechenden Lösungsmittels genommen. Nach der Probenahme wird das Reaktionsgemisch ca. 5 Minuten mittels Kanülen mit Stickstoff gespült (Raumtemperatur), danach wird der Katalysator (0.5 mol.%) zugegeben und das Reaktionsgefäß ins temperierte Ölbad plaziert und mittels eines Magnetrührers gerührt. Die Reaktion wird unter kontinuierlichem Spülen mit Stickstoff durchgeführt. Die Proben für GC-, GC-MS-und GPC-Messungen werden durch das Septum mittels einer Mikroliterspritze (10 µL, Hamilton bei Sigma-Aldrich, Art.-Nr. 21316) nach 5, 20, 30, 60 und 180 Minuten Reaktion genommen.

B) Isomerisierung ohne inerte Atmosphäre: Im Reaktionsglas wird das Gemisch aus 0.1 mL (0.49 mmol) trans-2-Undecen-1-ol und 0.02 mL Tetradecan (interner Standard) vorbereitet und gemischt. Wenn die Reaktion lösungsmittelfrei durchgeführt wird, wird eine Null-Probe für GC-Messung genommen. Bei der Durchführung mit Lösungsmittel wird eine Null-Probe für GC-Messung erst nach Zugabe der entsprechenden Lösungsmittel genommen. Nach der Probenahme wird der Katalysator (0.5 mol.%) zugegeben und das Reaktionsgefäß ins temperierte Ölbad plaziert und mittels eines Magnetrührers gerührt. Die Proben für GC- , GC- MS- und GPC-Messungen werden nach 5, 20, 30, 60 und 180 Minuten Reaktion genommen.

Probevorbereitung für GC-Messungen: In das 2 mL Probefläschchen für die GC Messungen wurden 10 - 30 µL Reaktionsgemisch und 100 µL Ethyl-vinylether zugegeben und mit 0.89 - 1.37 mL THF (HPLC Reinheitsgrad) verdünnt.

### Ausführungsbeispiele

### Beispiel 1:

1 mL (2.95 mmol) Ölsäuremethylester, 0.24 mL (2.95 mmol) 2-Buten-1,4-diol und 0.1 mL Tetradecan wurden im Reaktionsgefäß gemischt. Die erste GC-Probe wird genommen. Das Gemisch wird 2 Minuten bei 60 °C gerührt und 50 mg (0.059 mmol, 2 mol.%) Katalysator GII werden zugegeben. Das Gemisch wird weitere 24 Stunden bei 60 °C gerührt. Nach 4 und 24 Stunden Reaktion werden die nächsten GC-Proben genommen. Nach 24 Stunden wird die Reaktion durch Zugabe von 1 mL Ethyl-vinylether gestoppt. Umsatz von Ölsäuremethylester nach 24 Stunden: 64%. Ausbeute der Kreuzmetathese-Produkte: 27 % (davon 98.5% Aldehyde, 1.5% Alkohole); Ausbeute der Selbstmetathese-Produkte: 37%.

### Beispiel 2:

1 mL (2.95 mmol) Ölsäuremethylester, 0.73 mL (8.85 mmol) 2-Buten-1,4-diol, 0.1 mL Tetradecan und 1 mL 1-Butanol wurden im Reaktionsglas gemischt. Die erste GC-Probe wird genommen. Das Gemisch wird 2 Minuten bei 60 °C gerührt und 37 mg (0.059 mmol, 2 mol.%) Katalysator HGII werden zugegeben. Das Gemisch wird weitere 24 Stunden bei 60 °C gerührt. Nach 24 Stunden Reaktion wird das Reaktionsgemisch mit 1 mL 1-Butanol verdünnt und die nächste GC-Probe genommen. Danach wird die Reaktion durch Zugabe von 1 mL Ethyl-vinylether gestoppt. Umsatz von Ölsäuremethylester nach 24 Stunden: 82 %. Ausbeute der Kreuzmetathese-Produkte: 63 % (davon 6.5 % Aldehyde, 93.5 % Alkohole); Ausbeute der Selbstmetathese-Produkte: 19 %.

### Beispiel 3:

1 mL (2.95 mmol) Ölsäuremethylester, 0.73 mL (8.85 mmol) 2-Buten-1,4-diol, 0.1 mL Tetradecan und 1 mL 1-Butanol wurden im Reaktionsglas gemischt. Die erste GC-Probe wird genommen. Das Gemisch wird 2 Minuten bei 60 °C gerührt und 37 mg (0.059 mmol, 2 mol.%) Katalysator HGII werden zugegeben. Das Gemisch wird weitere 4 Stunden bei 60 °C gerührt, die nächste GC-Probe wird danach genommen. 12 mg (10 mol.%) Natriumhydrid werden zur Reaktionslösung zugegeben und weiter bei 60 °C gerührt. Nach 24 Stunden Reaktion wird die letzte GC-Probe für kinetische Bestimmungen gezogen und die Reaktion wird durch Zugabe von 1 mL Ethyl-vinylether gestoppt. Umsatz von Ölsäuremethylester nach 24 Stunden: 75 %. Ausbeute der Kreuzmetathese-Produkte: 58 % (davon 24 % Aldehyde, 76 % Alkohole); Ausbeute der Selbstmetathese-Produkte: 14%.

### Beispiel 4:

1 mL (2.95 mmol) Ölsäuremethylester, 0.24 mL (2.95 mmol) 2-Buten-1,4-diol, 0.1 mL Tetradecan und 1 mL o-Xylol wurden im Reaktionsglas gemischt. Die erste GC-Probe wird genommen. Das Gemisch wird 2 Minuten bei 60 °C gerührt und 49 mg (0.059 mmol, 2 mol.%) Katalysator GI werden zugegeben. Das Gemisch wird weitere 4 Stunden bei 60 °C gerührt, die nächste GC-Probe wird danach genommen. 70.3 µL (10 mol.%) Tributylamin werden zur Reaktionslösung zugegeben und weiter bei 60 °C gerührt. Nach 24 Stunden Reaktion wird die letzte GC-Probe für kinetische Bestimmungen gezogen und die Reaktion durch Zugabe von 1 mL Ethyl-vinylether gestoppt. Umsatz von Ölsäuremethylester nach 24 Stunden: 2.5 %. Ausbeute der Kreuzmetathese-Produkte: 1.5 %; Ausbeute der Selbstmetathese-Produkte: 1 %.

### Beispiel 5:

1 mL (2.95 mmol) Ölsäuremethylester, 0.73 mL (8.85 mmol) 2-Buten-1,4-diol und 0.1 mL Tetradecan wurden im Reaktionsglas gemischt. Die erste GC-Probe wird genommen. Das Gemisch wird 2 Minuten bei 120 °C gerührt und 50 mg (0.059 mmol, 2 mol.%) Katalysator GII werden zugegeben. Das Gemisch wird weitere 24 Stunden bei 120 °C gerührt. Nach 4 und 24 Stunden Reaktion werden die nächsten GC-Proben für kinetische Bestimmungen genommen. Nach 24 Stunden wird die Reaktion durch zugabe von 1 mL Ethyl-vinylether gestoppt. Umsatz von Ölsäuremethylester nach 24 Stunden: 67 %. Ausbeute der Kreuzmetathese-Produkte: 48%; Ausbeute der Selbstmetathese-Produkte: 19 %.

### Beispiel 6:

2 mL (5.9 mmol) Ölsäuremethylester, 2.43 mL (29.5 mmol) 2-Buten-1,4-diol, 0.2 mL Tetradecan und 2 mL 2-Propanol wurden im Reaktionsglas gemischt. Die erste GC-Probe wird genommen. Das Gemisch wird 2 Minuten bei 60 °C gerührt und 86.6 mg (0.118 mmol, 2 mol.%) 1,3-Bis-(2,4,6-trimethylphenyl)-4,5-dihydroimidazol-2-yliden[2-(i-propoxy)-5-N,N-dimethylaminosulfonyl)phenyl]methylenruthenium(II)dichlorid (Zannan Katalysator der Fa. ABCR) werden zugegeben. Das Gemisch wird weitere 4 Stunden bei 60 °C im Carousel-Reaktor gerührt. Nach 4 Stunden wird die letzte GC-Probe genommen und die Reaktion durch zugabe von 1 mL Ethyl-vinylether gestoppt. Umsatz von Ölsäuremethylester nach 4 Stunden: 65.5 %. Ausbeute der Kreuzmetathese-Produkte: 48.5 %; Ausbeute der Selbstmetathese-Produkte: 17 %.

### Beispiel 7:

2 mL (5.9 mmol) Ölsäuremethylester, 1.46 mL (17.7 mmol) 2-Buten-1,4-diol, 0.2 mL Tetradecan und 2 mL 2-Propanol wurden im Reaktionsglas gemischt. Die erste GC-Probe wird genommen. Das Gemisch wird 2 Minuten bei 40 °C gerührt und 100 mg (0.118 mmol, 2 mol.%) Katalysator GII werden zugegeben. Das Gemisch wird weitere 4 Stunden bei 40 °C im Carousel-Reaktor gerührt. Nach 4 Stunden wird die letzte GC-Probe genommen und die Reaktion durch Zugabe von 1 mL Ethyl-vinylether gestoppt. Umsatz von Ölsäuremethylester nach 4 Stunden: 85.5 %. Ausbeute der Kreuzmetathese-Produkte: 72 %; Ausbeute der Selbstmetathese-Produkte: 13.5%.

### Beispiel 8:

2 mL (5.9 mmol) Ölsäuremethylester, 1.46 mL (17.7 mmol) 2-Buten-1,4-diol, 0.2 mL Tetradecan und 2 mL THF wurden im Reaktionsglas gemischt. Die erste GC-Probe wird genommen. Das Gemisch wird 2 Minuten bei 40 °C gerührt und 100 mg (0.118 mmol, 2 mol.%) Katalysator GII werden zugegeben. Das Gemisch wird weitere 4 Stunden bei 40 °C im Carousel-Reaktor gerührt. Nach 4 Stunden wird die letzte GC-Probe genommen und die Reaktion durch Zugabe von 1 mL Ethyl-vinylether gestoppt. Umsatz von Ölsäuremethylester nach 4 Stunden: 65.5 %. Ausbeute der Kreuzmetathese-Produkte: 51.5 %; Ausbeute der Selbstmetathese-Produkte: 14 %.

### Beispiel 9:

Beispiel Zweistufenverfahren Im Reaktionsgefäß wird das Gemisch aus 0.1 mL (0.49 mmol) trans-2-Undecen-1-ol, 0.02 mL Tetradecan (interner Standard) und 2.9 mL 1,4-Dioxan vorbereitet, bei Raumtemperatur (= erste Temperatur) gemischt. Die ersten Proben für GC, GC-MS und GPC werden genommen. Danach werden 2.1 mg (0.0025 mmol, 0.5 mol.%) Katalysator GII bei Raumtemperatur (= erste Temperatur) zugegeben und eine Minute gerührt. Das Reaktionsgefäß wird dann in ein auf 100 °C (= zweite Temperatur) vorgeheiztes Ölbad gegeben, die Reaktionsmischung gerührt und innerhalb von zwei Minuten erreicht die Reaktionsmischung 100 °C (= zweite Temperatur). Anschließend wird die Reaktionsmischung 60 Minuten bei 100 °C (= zweite Temperatur) mittels eines Magnetrührers gerührt. Nach 5, 30 und 60 Minuten werden die Proben für GC-, GC-MS- und GPC-Messungen genommen und die Reaktion im Probengefäß mittels Zugabe von Ethyl-Vinyl Ether gestoppt. Umsatz von trans-2-Undecen-1-ol nach 60 Minuten: 94.0 %. Ausbeute des Undecanals (Produkt der Isomerisierung): 67.5 %; Ausbeute des Selbstmetathese-Produktes (9-Octadecen): 0 %; Nebenprodukte (Oligomere): 27 %.

Das Beispiel 9 wird bei unterschiedlichen Temperaturen, Konzentrationen und mit unterschiedlichen Katalysatoren wiederholt. Die Ergebnisse sind in den folgenden Tabellen zusammengefaßt:

**Tabelle 1:**

| Isomerisierung von trans-2-Undecen-1-ol in Butanol (Volumen Verhältnis 1 : 3), einer ersten Temperatur von 21 °C (Raumtemperatur) und unter Verwendung von G-II als Katalysator (0.5 %). Die zweite Temperatur T2 wird variiert. Daten nach 5 Minuten Reaktionszeit: | | | | |
|---|---|---|---|---|
| | Umsatz (in %) von trans-2-Undecen-1-ol | Ausbeute (in %) Undecanal | Ausbeute (in %) g-Octa-decen | Nebenprodukte (in %), z.B. Oligomere |
| T2 = 80 °C | 29 | 16 | 14 | < 2 |
| T2 = 100 °C | 60 | 18 | 7 | 35 |
| T2 = 120 °C | 99 | 64 | 14 | 22 |

Die Ergebnisse zeigen, dass bei Temperaturen ab 80 °C die Isomerisierung der hydroxyfunktionalen Verbindung deutlich zu beobachten ist. Bei Temperaturen von mehr als 100 °C werden bereits beachtliche Umsätze von mehr als 60% erhalten und es sind vermehrt Nebenreaktionen zu beobachten. Erst bei einer Temperatur von 120°C werden hohe Ausbeuten des Undecanals erhalten und der Anteil an Nebenprodukten wird reduziert.

### 3. Analytische Daten

GC-Messungen. Die Komponenten des Reaktionsgemisches wurden bei folgenden Retentionszeiten registriert: Undecanal (t_{R} = 7.34 min), 2-Undecen-1-ol (t_{R} = 11.54 min), 11-Hydroxy-9-undecensäuremethylester (t_{R} = 19.88 min), 11-Oxo-undecansäuremethylester (t_{R} = 15.90 min), 9-Octadecen (t_{R} = 10.29 min), 1,18-Dimethyl-9-octadecendicarbonsäure (t_{R} = 29.27 min), Tetradecan (t_{R} = 4.76 min), 2-Buten-1,4-diol (t_{R} = 13.16 min), Ölsäuremethylester (t_{R} = 18.09 min).

GC-MS (EI) Messungen. Die Komponenten des Reaktionsgemisches wurden bei folgenden Retentionszeiten registriert: Undecanal (t_{R} = 5.65 min), 2-Undecen-1-ol (t_{R} = 6.19 min), 11-Hydroxy-9-undecensäuremethylester (t_{R} = 8.88 min), 11-Oxo-undecansäuremethylester (t_{R} = 8.42 min), 9-Octadecen (t_{R} = 9.50 min), 1,18-Dimethyl-9-octadecendicarbonsäure (t_{R} = 16.78 min), Tetradecan (t_{R} = 6.44 min), 2-Buten-1,4-diol (t_{R} = 2.68 min), Ölsäuremethylester (t_{R} = 12.99 min).

Undecanal. GC-MS (EI, m/z, %): 170.9 (M^{+·}, 3), 123.0 (6), 108.9 (13), 94.9 (45), 81.8 (60), 67.0 (98), 55.0 (65), 41.0 (100). MS (ESI-positive, CH₃OH, m/z): 193.7 (MNa⁺, calc. 193.3).

2-Undecen-1-ol. GC-MS (EI, m/z, %): 151.9 (M^{+·}-H₂O, 5), 137.9 (2), 123.0 (10), 109.0 (23), 95.2 (100), 83.0 (75), 67.2 (28), 57.1 (22). MS (ESI-positive, CH₃OH, m/z): 193.7 (MNa⁺, calc. 193.3).

11-Hydroxy-9-undecensäuremethylester. GC-MS (EI, m/z, %): 196.8 (M^{+·}-H₂O, 20), 164.0 (22), 147.0 (28), 136.0 (25), 120.9 (25), 110.8 (30), 98.0 (70), 79.0 (45), 66.9 (67), 55.0 (100), 41.0 (70). MS (ESI-positive, CH₃OH, m/z): 237.1 (MNa⁺, calc. 237.3).

11-Oxo-undecansäuremethylester. GC-MS (EI, m/z, %): 214.8 (M^{+·}, 65), 183.0 (30), 171.0 (45), 164.9 (17), 139.0 (90), 121.0 (42), 111.0 (20), 97.0 (30), 80.9 (58), 73.9 (70), 66.9 (35), 55.0 (90), 43.0 (100). MS (ESI-positive, CH₃OH, m/z): 237.1 (MNa⁺, calc. 237.3).

9-Octadecen. GC-MS (EI, m/z, %): 252.0 (M^{+·}, 3), 208.0 (2), 193.9 (2), 180.0 (3), 166.0 (4), 152.0 (3), 138.0 (5), 124.9 (20), 110.9 (40), 97.0 (100), 82.8 (87), 57.0 (56), 55.1 (90).

1,18-Dimethyl-9-octadecendicarbonsäure. GC-MS (EI, m/z, %): 341.0 (M^{+·}, 6), 308.2 (52), 290.2 (16), 276.2 (100), 248.3 (26), 189.2 (7), 175.2 (9), 161.2 (14), 147.2 (38), 134.1 (36), 119.2 (20), 109.2 (25), 98.1 (48), 83.0 (73), 67.2 (77), 55.0 (98). MS (ESI-positive, CH₃OH, m/z): 363.3 (MNa⁺, calc. 363.5).

### 4. Ergebnisse

Die erfindungsgemäßen Beispiele belegen, dass
- die gewünschten Kreuzmetatheseprodukte als Gemisch aus Aldehyden und Alkoholen entstehen
- die entstehenden Produkte eindeutig nachgewiesen werden konnten
- die Reaktionsführung, und vor allem die Temperatur und das Lösungsmittel, einen großen Einfluss auf das Verhältnis zwischen den Aldehyd- und den Alkohol-Produkten hat
- die Reaktionsführung je nach Wahl der Reaktionsbedingungen einen großen Einfluss auf die Selektivität zwischen Kreuz- und Selbstmetathese hat, beispielsweise durch Verwendung eines geeigneten Lösungsmittels, Katalysators oder die Wahl einer geeigneten Temperatur zur Steuerung der Reaktion zugunsten der Selbstmetathese
- die entstehenden Alkohole bei höheren Temperaturen selektiver zu den entsprechenden Aldehyden isomerisiert werden können

## Patentansprüche

1. Verfahren zur Herstellung einer hydroxy- und aldehydfunktionale Verbindungen enthaltenden Mischung durch Kreuz-Metathesereaktion einer olefinischen Verbindung, die mindestens eine Hydroxygruppe und mindestens eine C-C-Doppelbindung aufweist, mit mindestens einer mindestens einfach ungesättigten Fettsäure oder mindestens einem mindestens einfach ungesättigten Fettsäurederivat in Gegenwart eines Metathesekatalysators bei einer Temperatur von höchstens 180 °C.

2. Verfahren nach Anspruch 1,
**dadurch gekennzeichnet,**
**dass** die olefinische Verbindung, die mindestens eine Hydroxygruppe und mindestens eine C-C-Doppelbindung aufweist, im Überschuss vorliegt - bezogen auf die mindestens einfach ungesättigte Fettsäure oder das mindestens einfach ungesättigte Fettsäurederivat.

3. Verfahren nach Anspruch 1 oder 2,
**dadurch gekennzeichnet,**
**dass** die Temperatur während Kreuz-Metathesereaktion zwischen 80 und 120 °C beträgt.

4. Verfahren nach Anspruch 1 oder 2,
**dadurch gekennzeichnet, dass** die Temperatur während Kreuz-Metathesereaktion zwischen 0 und 60 °C beträgt.

5. Verfahren nach Anspruch 1 oder 2,
**dadurch gekennzeichnet,**
**dass** die Kreuz-Metathesereaktion zuerst bei einer Temperatur zwischen 0 und 60 °C durchgeführt wird und die so erhaltene Mischung dann auf eine Temperatur zwischen 80 und 120 °C gebracht wird.

6. Verfahren nach einem der vorherigen Ansprüche,
**dadurch gekennzeichnet,**
**dass** die olefinische Verbindung mit mindestens einer Doppelbindung in einem Verhältnis von 1 bis 10, bevorzugt 2 bis 4, Äquivalenten (bezogen auf das mindestens einfach ungesättigte Fettsäurederivat) eingesetzt wird.

7. Verfahren nach einem der vorherigen Ansprüche,
**dadurch gekennzeichnet,**
**dass** die Kreuz-Metathesereaktion in Gegenwart mindestens eines Lösemittels durchgeführt wird.

8. Verfahren nach Anspruch 7,
**dadurch gekennzeichnet,**
**dass** das Lösemittel ein Dipolmoment zwischen 0 und 5 Debye aufweist.

9. Verfahren nach einem der vorherigen Ansprüche,
**dadurch gekennzeichnet,**
**dass** die Kreuz-Metathesereaktion in homogener Phase durchgeführt wird.

10. Verfahren nach einem der vorherigen Ansprüche,
**dadurch gekennzeichnet,**
**dass** die mindestens einfach ungesättigte Fettsäure ausgewählt ist aus der Gruppe von Ölsäure, Undecylensäure, Palmitoleinsäure, Petroselinsäure, Erucasäure, Icosenäure, Vernolsäure, Ricinolsäure, Linolsäure, Linolensäure.

11. Verfahren nach einem der vorherigen Ansprüche,
**dadurch gekennzeichnet,**
**dass** das mindestens einfach ungesättigte Fettsäurederivat ausgewählt ist aus der Gruppe der Ester von Säuren nach Anspruch 11 mit Methanol, Ethanol, Propanol, Gycerin, und/oder Glykol.

12. Verfahren nach einem der vorherigen Ansprüche,
**dadurch gekennzeichnet,**
**dass** die olefinische Verbindung ausgewählt ist aus der Gruppe von Allylalkohol, But-3-en-1-ol, und 1,4-But-2-en-diol.
